# EUROPÄISCHE PATENTSCHRIFT

(11) **EP 3 062 681 B1**
(45) Veröffentlichungstag und Bekanntmachung des Hinweises auf die Patenterteilung: **06.06.2018**
(21) Anmeldenummer: 14789842.3
(22) Anmeldetag: 28.10.2014
(51) Int. Cl.: A61B 1/012

(54) **AN EIN MUTTERENDOSKOP ANBRINGBARES SEKUNDÄRENDOSKOP UND KOMBINATION AUS MUTTERENDOSKOP UND SEKUNDÄRENDOSKOP**
SECONDARY ENDOSCOPE WHICH CAN BE ATTACHED TO A PARENT ENDOSCOPE AND COMBINATION OF PARENT ENDOSCOPE AND SECONDARY ENDOSCOPE
ENDOSCOPE SECONDAIRE POUVANT SE MONTER SUR UN ENDOSCOPE PRINCIPAL ET COMBINAISON D'UN ENDOSCOPE PRINCIPAL ET D'UN ENDOSCOPE SECONDAIRE

(30) Priorität: 30.10.2013 DE 102013222039
(43) Veröffentlichungstag der Anmeldung: 07.09.2016
(73) Patentinhaber: Digital Endoscopy GmbH, 86316 Friedberg (DE)
(72) Erfinder: VIEBACH, Thomas, 86579 Waidhofen (DE); PAUKER, Friedrich, 86420 Diedorf (DE)
(74) Vertreter: TBK
(86) Internationale Anmeldenummer: PCT/EP2014/073064
(87) Internationale Veröffentlichungsnummer: WO 2015/063051

(56) Entgegenhaltungen:
- WO-A2-2005/094665
- JP-A- 2007 111 541
- US-A1- 2006 252 993

## Beschreibung

Die vorliegende Erfindung bezieht sich auf ein an ein Mutterendoskop anbringbares Sekundärendoskop. Die vorliegende Erfindung bezieht sich außerdem auf eine Kombination aus einem Mutterendoskop und einem Sekundärendoskop.

Bei einem Endoskop wird ein biegbares Ende eines Katheters, das heißt ein sogenannter Deflectingabschnitt, durch Schwenken eines Steuerelementes bewegt, wobei die Bewegung des Deflectingabschnittes genau der Bewegung des Steuerelementes folgt.

Bei medizinischen Untersuchungen mit einem Endoskop sollte die Übertragung einer Schwenkbewegung eines Steuerelementes zu einer Biegebewegung des Deflectingabschnittes möglichst genau sein. Andererseits sollte die Übertragung einer Schwenkbewegung eines Steuerelementes zu einer Biegebewegung des Deflectingabschnittes für den Anwender einfach und übersichtlich ausführbar sein. Eine Vorrichtung mit den Merkmalen des Oberbegriffs des Anspruchs 1 ist dem Dokument US 2006/252993 zu entnehmen.

### DURCH DIE ERFINDUNG ZU LÖSENDE AUFGABE

Aufgabe der vorliegenden Erfindung ist es, ein verbessertes an ein Mutterendoskop anbringbares Sekundärendoskop zu schaffen.

Insbesondere ist es die Aufgabe der vorliegenden Erfindung, ein Sekundärendoskop mit besonders günstiger Funktionalität und einfacher Bedienbarkeit zu schaffen, bei dem die Handhabung bei der Schwenkbewegung des Steuerelementes verbessert ist. Außerdem soll eine verbesserte Kombination aus einem Mutterendoskop und einem Sekundärendoskop geschaffen werden.

### LÖSUNG DER AUFGABE

Diese Aufgabe wird erfindungsgemäß durch ein Sekundärendoskop mit den Merkmalen von Anspruch 1 gelöst.

Eine Kombination aus einem Mutterendoskop und einem Sekundärendoskop ist in Anspruch 11 aufgezeigt. Eine alternative Kombination aus einem Mutterendoskop und einem Sekundärendoskop ist in Anspruch 12 aufgezeigt.

Vorteilhafte Weiterbildungen sind Gegenstand der abhängigen Ansprüche.

Die Erfindung betrifft somit ein an ein Mutterendoskop anbringbares Sekundärendoskop mit einem Griffkörper; einem am Griffkörper angebrachten Katheter; einem Steuerelement an der proximalen Seite des Griffkörpers zur Steuerung eines am distalen Ende des Katheters angeordneten Deflectingabschnittes (Biegeabschnitt); und einem Montageanschluss an der distalen Seite des Griffkörpers zum Befestigen des Sekundärendoskops an einem Mutterendoskop.

Der Griffkörper als Basiselement des Sekundärendoskops besitzt an der proximalen Seite das Steuerelement und an der distalen Seite den Montageanschluss zum Mutterendoskop. Das Steuerelement ist vom Anwender leicht bedienbar, da es sich ihm entgegenstreckt.

Der Griffkörper kann einen Katheterkanal zur Führung des Katheters aufweisen. Der Katheterkanal kann ein Innenkanal im Inneren des Griffkörpers sein. In diesem Sekundärendoskop ist der Katheter samt Deflectingabschnitt durch das Steuerelement, das die Auslenkbewegung des Deflectingabschnittes steuert, hindurchsteckbar. Somit wird ein Sekundärendoskop geschaffen, das auf engstem Raum eine sichere und übersichtliche Handhabung einer Schwenkbewegung eines Deflectingabschnittes ermöglicht. Der Innenkanal kann konzentrisch im Griffkörper angeordnet sein. Die Stabilität der gesamten Vorrichtung wird dadurch nicht beeinträchtigt.

Das proximale Ende des Steuerelementes kann eine proximale Eingangsöffnung des Katheterkanals aufweisen. Darüber hinaus kann die proximale Eingangsöffnung des Katheterkanals im Griffkörper eine trichterartige Einführöffnung als Einführhilfe aufweisen. Eine sichere Führung des hindurchzusteckenden Katheters wird dadurch ermöglicht.

Das proximale Ende des Katheters kann an der distalen Seite des Griffkörpers angebracht sein.

Das proximale Ende des Katheters kann an der Außenseite des Griffkörpers angebracht sein. Der Weg vom Befestigungspunkt des Katheters am Griffkörper bis zur proximalen Eingangsöffnung des Katheterkanals kann somit kurz gestaltet sein, wobei der Griffkörper als solches kurz gebaut sein kann, selbst wenn sich ein Katheteranschlussabschnitt am Griffkörper in distaler Richtung erstreckt.

Das distale Ende des Griffkörpers kann einen Montagekörper zur Montage am Mutterendoskop aufweisen, wobei der Montagekörper einen Abschnitt des Katheterkanals zur Führung des Katheters aufweist.

Der Montagekörper kann mit einer Markierung versehen sein, die über den montierten oder demontierten Zustand informiert.

Der Montagekörper kann ein Luer-lock-Verbindungselement, ein Schraubverschlusselement, ein Bajonettverschlusselement oder ein Einrastverbindungselement sein.

Das Steuerelement kann zum Schwenken des Deflectingabschnittes schwenkbar sein und an seiner proximalen Seite mit Markierungen versehen sein, die über die Schwenkrichtung des Deflectingabschnittes informieren. Das Steuerelement ist somit ein Schwenkabschnitt, der an einem Kopfabschnitt des Basiskörpers abgestützt ist und zur Bewerkstelligung einer Auslenkbewegung relativ zum Kopfabschnitt des Basiskörpers schwenkbar ist. Das Steuerelement ist somit schwenkbar und durch seine Schwenkbewegung wird der Deflectingabschnitt gesteuert. Das Steuerelement kann arretierbar sein, um eine Auslenkstellung des Steuerelements zu arretieren. Das Steuerelement kann ein Joystick sein.

Eine erfindungsgemäße Kombination aus Mutterendoskop und Sekundärendoskop ist versehen mit einem Mutterendoskop mit einem Zugangsanschluss für ein Sekundärendoskop an der distalen Seite eines Mutterendoskopgriffes; und einem am Zugangsanschluß lösbar montierten Sekundärendoskop mit einem Sekundärendoskopgriff und einem Katheter; wobei der Katheter des Sekundärendoskops durch einen Katheterkanal des Sekundärendoskopgriffs geführt ist; wobei das distale Ende des Sekundärendoskopgriffs am Zugangsanschluss des Mutterendoskops lösbar montiert ist; wobei in der montierten Position das Sekundärendoskop am Mutterendoskopgriff an der proximalen Seite des Zugangsanschlusses des Mutterendoskops lösbar montiert ist; wobei am Sekundärendoskop eine Kathetereingangsöffnung proximal vom Montageort des Sekundärendoskops positioniert ist.

Der Sekundärendoskopgriff kann des Weiteren eine Zusatzkanaleingangsöffnung an der proximalen Seite, und eine Fluideingangsöffnung an der distalen Seite aufweisen.

Die Merkmale der Erfindung können geeignet kombiniert werden.

Nachstehend ist die Erfindung detailliert anhand von Beispielen erläutert.

### Kurzbeschreibung der Zeichnungen

Figur 1 zeigt eine schematische Schnittansicht eines Sekundärendoskops eines ersten Ausführungsbeispiels im nicht ausgelenkten Zustand.
Figur 2 zeigt eine schematische Schnittansicht des Sekundärendoskops bei einer Auslenkung nach links.
Figur 3 zeigt eine schematische Schnittansicht des Sekundärendoskops bei einer Auslenkung nach rechts.
Figur 4 zeigt in einer schematischen perspektivischen ausschnittartigen Darstellung Einzelheiten der Anbindung der Drahtkörper am Steuerelement und wie die Drahtkörper zum Katheterschlauch geführt werden.
Figur 5 zeigt eine schematische Schnittdarstellung des Sekundärendoskops des ersten Ausführungsbeispiels, wobei der Katheterschlauch durch das Sekundärendoskop geführt ist und nicht ausgelenkt ist.
Figur 6 zeigt eine schematische Schnittdarstellung des Sekundärendoskops, wobei der Katheterschlauch durch das Sekundärendoskop geführt ist und eine Auslenkung nach links erfolgt ist.
Figur 7 zeigt eine schematische Schnittdarstellung des Sekundärendoskops, wobei der Katheterschlauch durch das Sekundärendoskop geführt ist und eine Auslenkung nach rechts erfolgt ist.

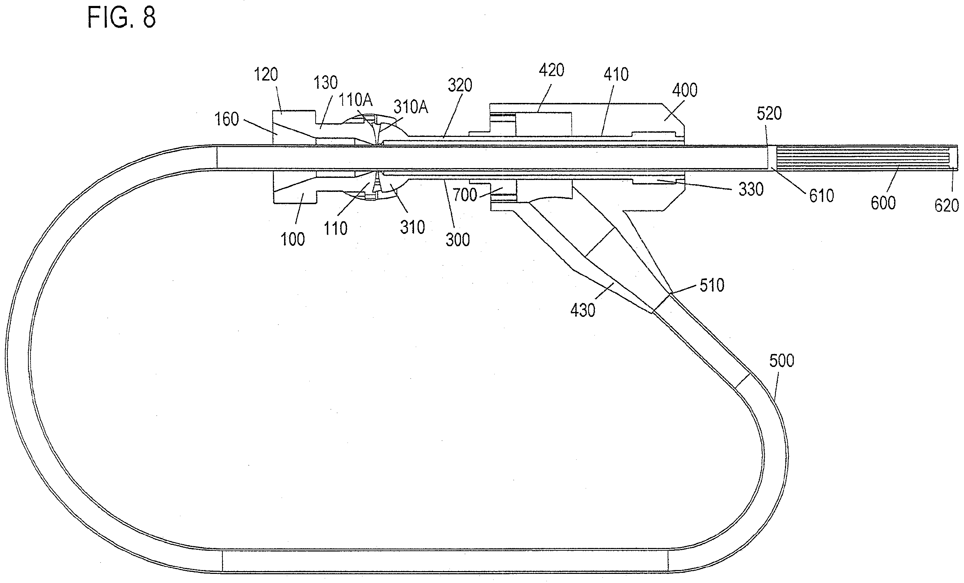

Figur 8 zeigt eine schematische Schnittansicht eines Sekundärendoskops eines zweiten Ausführungsbeispiels, wobei der Katheterschlauch durch das Sekundärendoskop geführt ist und nicht ausgelenkt ist.
Figur 9 zeigt eine schematische Schnittdarstellung des Sekundärendoskops des zweiten Ausführungsbeispiels, wobei der Katheterschlauch durch das Sekundärendoskop geführt ist und eine Auslenkung nach links erfolgt ist.
Figur 10 zeigt eine schematische Schnittdarstellung des Sekundärendoskops des zweiten Ausführungsbeispiels, wobei der Katheterschlauch durch das Sekundärendoskop geführt ist und eine Auslenkung nach rechts erfolgt ist.
Figur 11 zeigt eine schematische Schnittdarstellung des Sekundärendoskops des zweiten Ausführungsbeispiels, wobei an der distalen Seite des Griffkörpers ein Montagekörper zur Montage am Mutterendoskop sitzt.
Figur 12 zeigt eine schematische Darstellung im Teilschnitt einer Kombination aus einem Mutterendoskop und dem erfindungsgemäßen Sekundärendoskop.

Nachstehend sind Ausführungsbeispiele der vorliegenden Erfindung detailliert anhand der Zeichnungen beschrieben.

### Erstes Ausführungsbeispiel

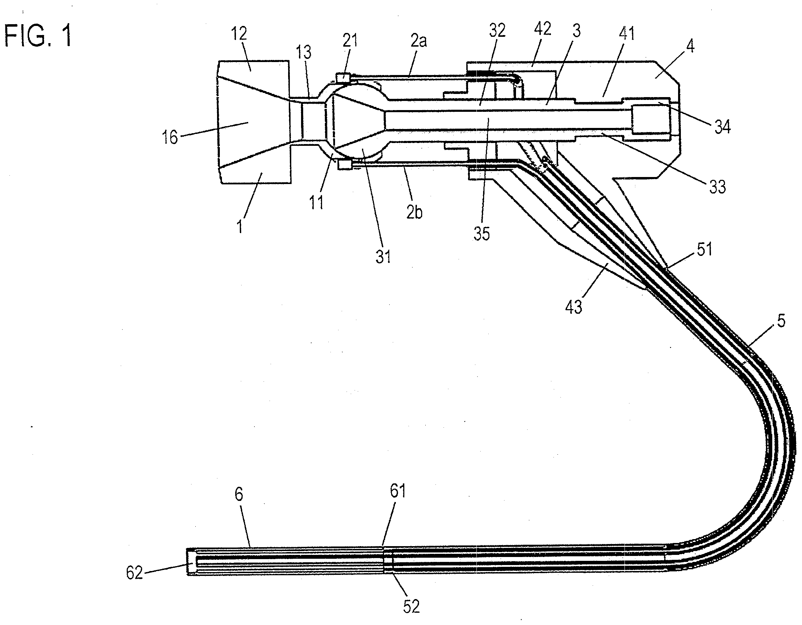

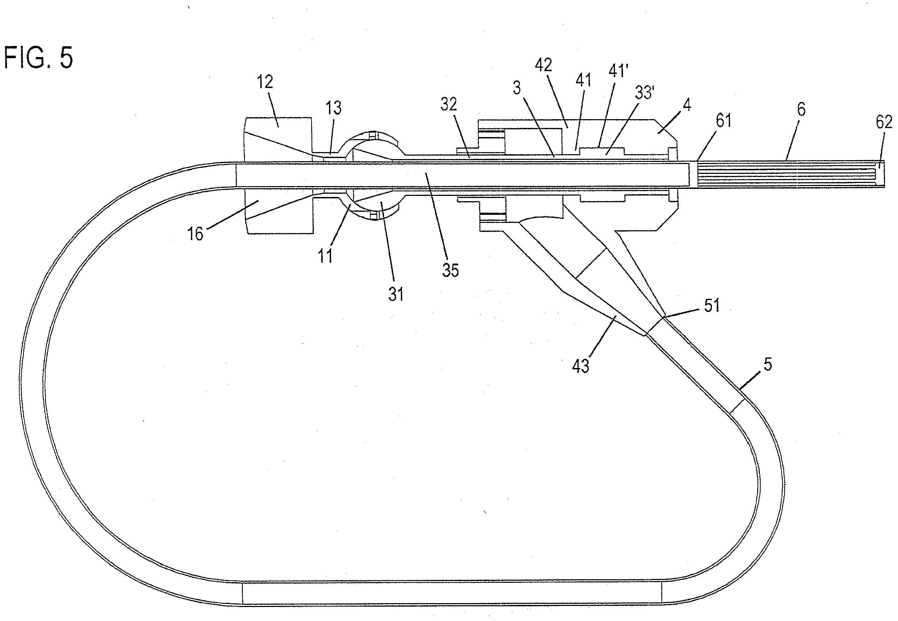

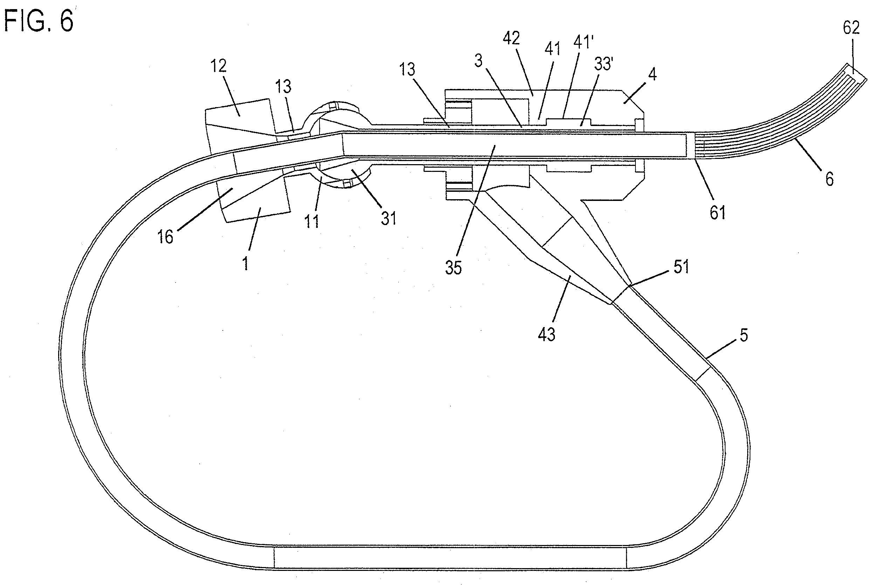

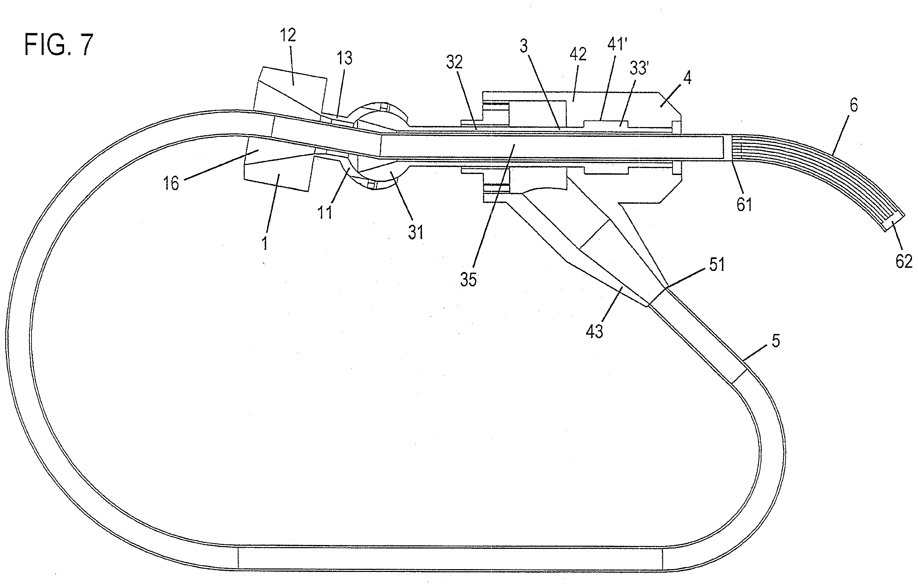

Zunächst ist ein erstes Ausführungsbeispiel der vorliegenden Erfindung detailliert anhand der Figuren 1 - 7 beschrieben.

Das erste Ausführungsbeispiel zeigt ein erfindungsgemäßes Sekundärendoskop, bei dem eine Endoskopdeflectingsteuerung durch ein Steuerelement bewerkstelligt wird.

Dieses Sekundärendoskop besteht im vorliegenden Ausführungsbeispiel aus einem Steuerelement 1, mehreren Drahtkörpern 2, einem Stabelement 3 als Basiselement, einem Stabelementhalter 4, einem Katheterschlauch 5 und einem Deflectingabschnitt 6.

Das Steuerelement 1 besteht aus einem hohlen zylindrischen Element mit einem Steuerkopf 12, an dessen Unterseite ein Hohlstab 13 zentrisch angeordnet ist, der in einen Hohlkugelabschnitt 11 übergeht, an dessen Außenfläche die Drahtkörper 2 verankert sind. Der Hohlkugelabschnitt 11 ist an seiner zum Kopf 12 abgewandten Seite hin offen. Insbesondere ist die Öffnung am Hohlkugelabschnitt 11 derart, dass der Hohlkugelabschnitt 11 zirka 9/10 einer Kugel ausmacht von der zirka 1/10 abgeschnitten ist.

Das Steuerelement 1 ist rotationssymmetrisch aufgebaut und weist einen Innenkanal 16 auf, der sich durch den Kopf 12, den Hohlstab 13 und den Hohlkugelabschnitt 11 konzentrisch erstreckt. Der Innenkanal 16 ist im Kopf 12 derart erweitert, dass sein Innendurchmesser zu der vom Hohlkugelabschnitt 11 abgewandten Seite des Kopfes 12 zunimmt, wie dies in Figur 1 gezeigt ist. Somit hat der Innenkanal 16 des Kopfes 12 eine trichterartige Eingangsöffnung, die in Fig. 1 an der linken Seite des Innenkanals 16 dargestellt ist. Die trichterartige Eingangsöffnung des Innenkanals 16 erleichtert es, den Deflectingabschnitt 6 in den Innenkanal 16 einzuführen.

Das Steuerelement 1 ist aus einem Kunststoffmaterial hergestellt.

Das Steuerelement 1 sitzt als ein Joystick auf einem Kopf 31 des Stabelementes 3. Insbesondere sitzt der Hohlkugelabschnitt 11 des Steuerelementes 1 auf einem Gegenkugelabschnitt 31, der den Kopf des Stabelementes 3 bildet. Der Gegenkugelabschnitt 31 ist dabei derart ausgebildet, dass er eine Kugelform in einer derartigen Größe hat, dass der darauf sitzende Hohlkugelabschnitt 11 leichtgängig bewegbar ist. Die Maßbeziehung zwischen dem Gegenkugelabschnitt 31 und dem Hohlkugelabschnitt 11 sind dabei derart, dass eine Relativbewegung des Steuerelementes 1 zum Stabelement 3 ohne große Kraftanstrengung des Anwenders möglich ist, andererseits aber der Hohlkugelabschnitt 11 nicht lose auf dem Gegenkugelabschnitt 31 sitzt.

Das Stabelement 3 besitzt einen Längszylinder 32, der an seiner distalen Seite in den Gegenkugelabschnitt 31 übergeht und an seinem distalen Endabschnitt ein Schraubende 34 aufweist, das im vorliegenden Ausführungsbeispiel als Innenvierkant ausgearbeitet ist. Distal von dem Vierkantende 34 besitzt das Stabelement 3 an seiner Außenzylinderfläche einen Außengewindeabschnitt 33. Das Stabelement 3 ist rotationssymmetrisch aufgebaut und besitzt in seinem Inneren einen Innenkanal 35, der konzentrisch durch den Gegenkugelabschnitt 31 den Längszylinder 32 und das Vierkantende 34 läuft. Im Übrigen sind der Gegenkugelabschnitt 31, der Längszylinder 32 und das Vierkantende 34 als ein einstückiges Stabelement aufgebaut. Der Längszylinder 32 des Stabelementes 3 ist mit Ausnahme des an ihn vorgesehenen Gewindeabschnittes 33 als ein Zylinder mit einer glatten Außenoberfläche ausgebildet.

Am Gegenkugelabschnitt 31 besitzt der Innenkanal 35 eine trichterartige Eingangsöffnung, die in Fig. 1 an der linken Seite des Innenkanals 35 dargestellt ist. Die trichterartige Eingangsöffnung des Innenkanals 35 steht der Ausgangsöffnung des Innenkanals 16 am Hohlkugelabschnitt 11 gegenüber und erleichtert es, den Deflectingabschnitt 6 in den Innenkanal 35 einzuführen.

Wie dies in den Figuren gezeigt ist, sitzt das Stabelement 3 in einem Stabelementhalter 4. Der Stabelementhalter 4 bildet den Griffkörper des Sekundärendoskops und besteht aus einem Zylinderelement 42, das rotationssymmetrisch aufgebaut ist und einen zentrischen Innenkanal aufweist. Das Zylinderelement 42 weist insbesondere einen zum Steuerelement 1 hinweisenden Hohlraum und einen Boden an der vom Steuerelement 1 abgewandten Seite des Stabelementhalters 4 auf. Genauer gesagt weist der Boden des Stabelementhalters 4 den konzentrischen Innenkanal auf. In dem konzentrischen Innenkanal ist eine Innengewinde 41 ausgearbeitet. Wie dies in den Figuren schematisch angedeutet ist, sitzt das Außengewinde 33 des Stabelementes 3 an dem Innengewinde 41 des Stabelementhalters 4, wobei durch eine Schraubbewegung das Stabelement 3 relativ zum Stabelementhalter 4 konzentrisch hinein oder herausgeschraubt werden kann. Zum Zwecke der Ausführung der Schraubbewegung wird ein entsprechendes Werkzeug in das Vierkantende 34 des Stabelementes 3 eingesetzt. Andere Relativbewegungstechniken sind möglich, wie dies unter "Alternativen" am Ende der Beschreibung dargestellt ist.

An seiner Außenumfangsseite besitzt das Zylinderelement 42 des Stabelementhalters 4 ein Katheteranschlusselement 43. Im vorliegenden Ausführungsbeispiel erstreckt sich das Katheteranschlusselement 43 unter einem spitzen Winkel relativ zu dem Zylinderelement 42 des Stabelementes 4, wie dies aus den Zeichnungen hervorgeht.

Insbesondere ist das Katheteranschlusselement 43 als ein rundes Hohlprofil ausgearbeitet, das quasi eine Kanalabzweigung aus dem distalen Hohlraum des Zylinderelementes 42 darstellt. Das Katheteranschlusselement 43 ist zylinderartig ausgebildet, wobei es sich in Richtung vom Zylinderelement 42 weg verjüngt. Im Inneren besitzt das Katheteranschlusselement 43 einen konzentrischen Kanal, in welchem die Drahtkörper 2 geführt werden. An seinem distalen Ende besitzt das Katheteranschlusselement 43 eine kreisartige Mündung.

An der kreisartigen Mündung des Katheteranschlusselementes 43 ist der Katheterschlauch 5 befestigt. Insbesondere sitzt das proximale Ende 51 des Katheterschlauches 5 an der Mündung des Katheteranschlusselementes 43. An seinem distalen Ende besitzt der Katheterschlauch einen in ihm aufgenommenen Ring 52. Der Ring 52 bildet das distale Ende des Katheterschlauches und den Übergang zu dem Deflectingabschnitt 6.

Der Deflectingabschnitt 6 ist ein biegbarer Körper, der in bekannterweise aus einem elastischem Material hergestellt ist. An seinem proximalen Ende besitzt der Deflectingabschnitt einen Deflectinganschluss 61, an dem er an dem Ring 52 des Katheterschlauches 5 angeschlossen ist. An dem distalen Ende besitzt der Deflectingabschnitt eine Deflectingkappe 62, an der eine Kamera, ein Laser und/oder eine Kamera etc. angeordnet sind. Weitere Funktionseinheiten können an der Deflectingkappe 62 integriert werden.

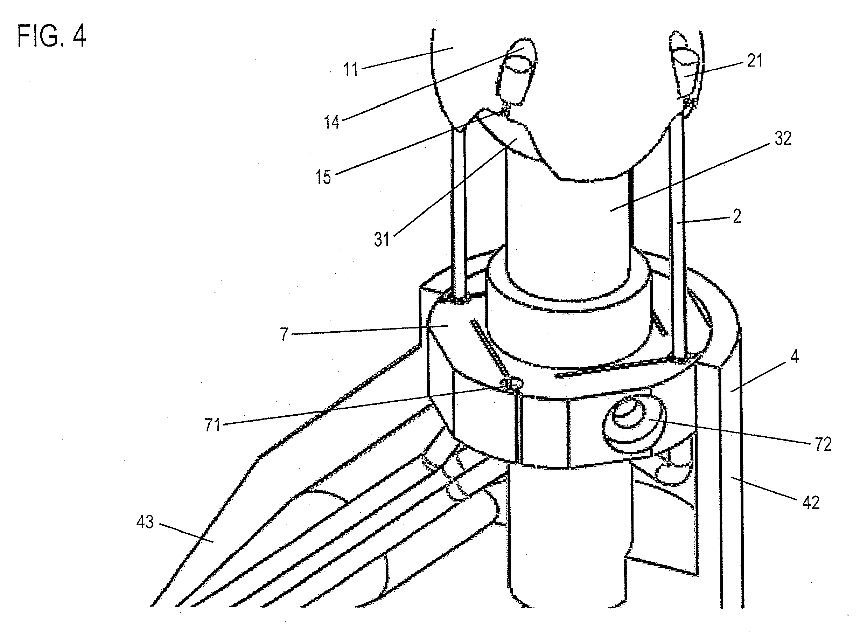

Figur 4 zeigt in einer schematischen perspektivischen ausschnittartigen Darstellung Einzelheiten der Anbindung der Drahtkörper am Steuerelement und wie die Drahtkörper zum Katheterschlauch geführt werden. Der besseren Übersichtlichkeit wegen ist in Fig. 4 der vordere linke Drahtkörper 2 weggelassen worden.

Wie dies in Figur 4 gezeigt ist, sind an der Außenumfangsfläche des Hohlkugelabschnittes 11 an der Äquatorlinie des Hohlkugelabschnittes 11 mehrere Einhängausbauchungen 14 vorgesehen. Im vorliegenden Ausführungsbeispiel sind vier Einhängausbauchungen 14 am Äquator des Hohlkugelabschnittes 11 vorgesehen. Insbesondere sind die Einhängausbauchungen 14 am Hohlkugelabschnitt 11 eingeformte im Querschnitt kreisartige Vertiefungen mit einem Boden, wobei der Boden ungefähr senkrecht zu einer Bohrungsrichtung der Einhängausbauchung 14 verläuft und sich an der Äquatorlinie genauer gesagt senkrecht zum Äquator des Hohlkugelabschnittes 11 befinden. Bei der Herstellung der Einhängausbauchung 14 kann der Hohlkugelabschnitt 11 von der proximalen Seite aus gebohrt werden, sodass die Einhängausbauchung als ein seitlich offenes Sackloch an der Außenumfangsfläche des Hohlkugelabschnittes 11 entsteht. Beliebige andere Herstellverfahren sind hierbei denkbar. Der Außendurchmesser der Einhängausbauchung 14 ist derart gewählt, dass ein Tonnennippel 21 des Drahtkörpers 2 in die Einhängausbauchung 14 hineinpasst. Am Boden der Einhängausbauchung 14, das heißt an dem distalen Ende der Einhängausbauchung 14 ist ein Kanal 15 als Drahtkörpereinhängung ausgearbeitet, der sich koaxial zu der Längserstreckung des Steuerelementes 1 erstreckt und einen Durchmesser hat, der größer als der Außendurchmesser des Drahtkörpers 2 ist, aber kleiner als der Außendurchmesser des Tonnennippels 21 des Drahtes 2 ist. Anders ausgedrückt sind die Einhängausbauchung 14 und die Drahtkörpereinhängung 15 ähnlich wie Baudenzugeinhängungen am Fahrrad so vorgesehen, dass ein Tonnennippel 21 eines Drahtkörpers 2 darin eingehängt werden kann. Das Tonnennippel bildet im eingehängten Zustand des Drahtkörpers 2 das proximale Ende des Drahtkörpers 2.

Im vorliegenden Ausführungsbeispiel sind vier Drahtkörper 2 vorgesehen, von denen in den Figuren 1 bis 3 jeweils zwei Drahtkörper, das heißt der Drahtkörper 2a und der Drahtkörper 2b, dargestellt sind. Die Anzahl an Drahtkörpern 2 ist hierbei nicht begrenzt. Es kann ein Drahtkörper 2, 2, 3, 4 oder mehr Drahtkörper vorgesehen sein. Sollten zwei oder mehr Drahtkörper 2 vorgesehen sein, sind die entsprechenden Einhängausbauchungen 14 gleichmäßig zueinander beabstandet am Äquator des Hohlkugelabschnittes 11 angeordnet.

Wie dies in Figur 4 gezeigt ist, besitzt das Zylinderelement 42 an seinem proximalen Ende das heißt an seinem zum Steuerelement 1 weisenden Ende die Öffnung zum Zylinderelementhohlraum. In diese Öffnung ist ein Drahtkörperführungsring 7 so eingesetzt, dass die proximale Fläche, das heißt die zum Steuerelement 1 weisende Fläche des Drahtführungsringes 7 zu der proximalen, das heißt zum Steuerelement 1 weisenden, Stirnfläche des Zylinderelementes 42 fluchtet. Der Drahtführungsring 7 ist mit tangentialen Schlitzen in gleicher Anzahl, wie Drahtführungen 2 vorhanden sind, versehen, wie dies in Figur 4 gezeigt ist. In den Schlitzen sind Drahtführungsöffnungen 71 gebohrt, die koaxial zu der gemeinsamen Achse des Steuerelementes 1, Stabelementes 4 und des Zylinderelementes 42 des Stabelementhalters 4 verlaufen. Genauer gesagt beträgt der Abstand jeder Einhängausbauchung 14 zu der Mittelachse des Steuerelementes 1 genauso viel wie der radiale Abstand zwischen der Drahtführungsbohrung 71 und der Mittelachse des Drahtführungsringes 7.

Die Drahtkörper 2 werden durch den Katheterschlauch 5 und durch den Ring 52 des Katheterschlauches hindurchgeführt und sind an der Deflectingkappe 62 des Deflectingabschnittes 6 verankert. Insbesondere sind die Drahtkörper 2 so an der Deflectingkappe 62 verankert, dass sie gleichmäßig zueinander beabstandet und in der gleichen Reihenfolge wie am Hohlkugelabschnitt 11 angeordnet, das heißt befestigt sind.

Der Ring 52 weist Öffnungen für die Drahtkörper 2 in entsprechender Weise wie bei der Ausgestaltung des Drahtführungsringes 7 auf.

### Funktionsweise

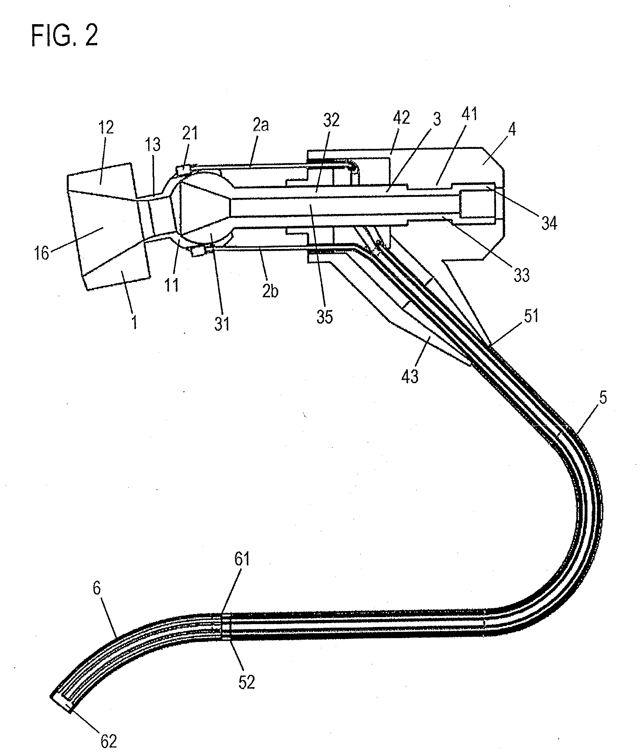

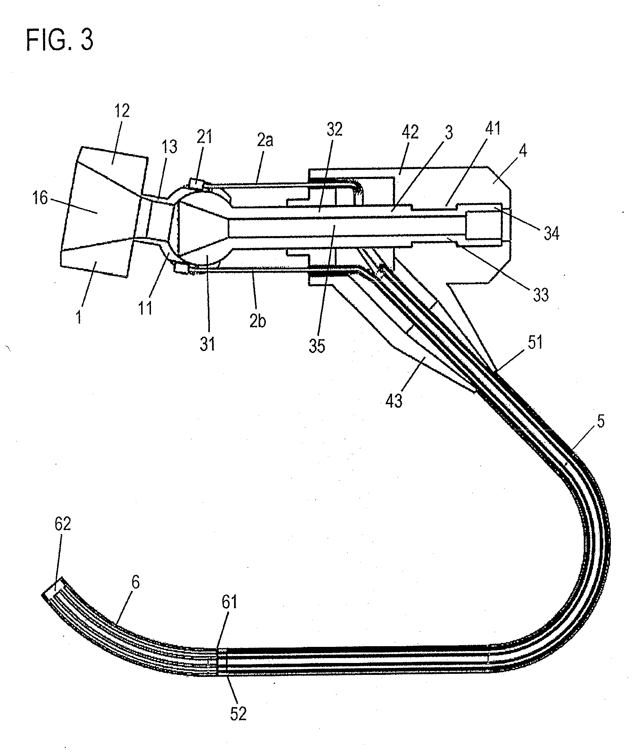

Das Steuerelement 1 ist als Joystick aufgebaut und kann wie ein solcher betätigt werden, wobei sein Hohlkugelabschnitt 11 auf dem Gegenkugelabschnitt 31 des Stabelementes 3 bewegt werden kann. Ein Schwenkvorgang des Joysticks 1 relativ zum Stabelement 3 in beliebiger Richtung ist dadurch möglich. Die Richtung und das Ausmaß der Auslenkbewegung des Joysticks 1 relativ zum Stabelement 3 wird dann durch die an der Deflectingkappe 62 angeordneten Drahtkörper 2 auf den als biegbaren Körper ausgestalteten Deflectingabschnitt 6 übertragen. Anders ausgedrückt, wenn der Joystick 1 relativ zum Stabelement 3 nach links bewegt wird, vollführt der Deflectingabschnitt eine nach links gerichtete Bewegung, wie dies in Figur 2 gezeigt ist. Wenn der Joystick 1 relativ zum Stabelement 3 nach rechts bewegt wird, vollführt der Deflectingabschnitt eine nach rechts gerichtete Bewegung, wie dies in Figur 3 der Fall ist.

Das Steuerelement 1 wird vor dem Einführen des Deflectingabschnitts 6 gerade gestellt, so dass der Deflectingabschnitt 6 und der am Deflectingabschnitt 6 angrenzende Abschnitt des Katheterschlauches 5 gerade ausgerichtet sind. Das distale Ende (an der Deflectingkappe 62) des Deflectingabschnitts 6 wird in die trichterartige Eingangsöffnung des Innenkanals 16 im Steuerelement 1 eingeführt, durch den Innenkanal 16 geschoben, in die trichterartige Eingangsöffnung des Innenkanals 35 im Stabelement 3 eingeführt und durch den Innenkanal 35 hindurch geschoben, bis der Deflectingabschnitt 6 an der zu der trichterartigen Eingangsöffnung des Innenkanals 35 entgegengesetzten Ausgangsöffnung des Innenkanals 35 heraustritt.

Wenn der Deflectingabschnitt 6 seine beabsichtigte durch das Sekundärendoskop hindurchgeschobene Betriebsposition erreicht hat, kann durch in der erwünschten Richtung und um das erwünschte Maß erfolgendes Schwenken des Steuerelementes 1 der Deflectingabschnitt 6 in die erwünschte Schwenkstellung gebracht werden. Das Steuerelement 1 als Joystick kann in alle Richtungen schwenken und somit kann der Deflectingabschnitt 6 nicht nur nach rechts und links, sondern in alle Richtungen schwenken.

### Zweites Ausführungsbeispiel

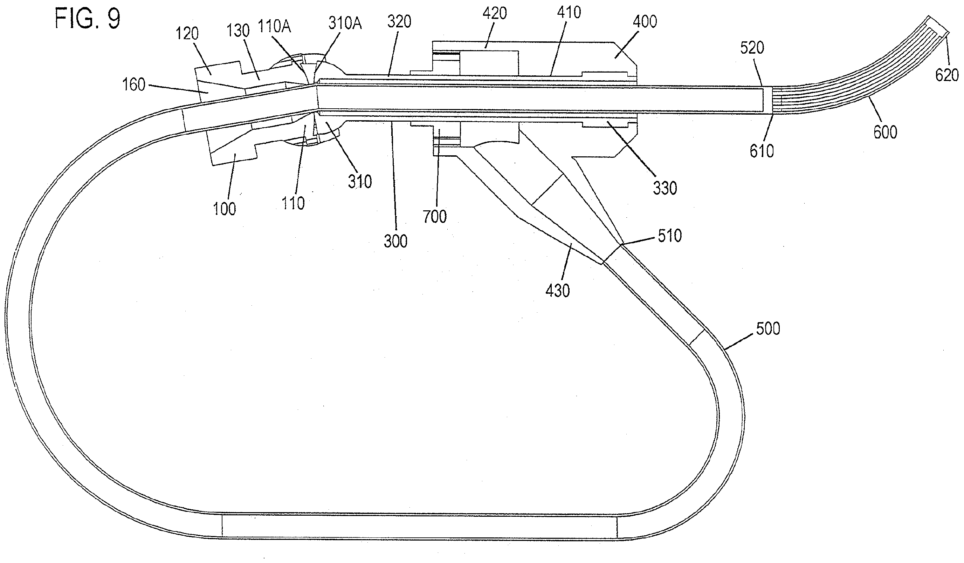

Nachstehend ist ein zweites Ausführungsbeispiel der vorliegenden Erfindung detailliert anhand der Figuren 9 - 11 beschrieben.

Das zweite Ausführungsbeispiel zeigt ein Sekundärendoskop, bei dem eine Endoskopdeflectingsteuerung durch ein Steuerelement bewerkstelligt wird.

Dieses Sekundärendoskop besteht auch im vorliegenden Ausführungsbeispiel aus einem Steuerelement 100, mehreren Drahtkörpern (in den Zeichnungen nicht dargestellt) in ähnlicher Weise wie im ersten Ausführungsbeispiel, einem Stabelement 300 als Basiselement, einem Stabelementhalter 400, einem Katheterschlauch 500 und einem Deflectingabschnitt 600.

Das Steuerelement 100 besteht aus einem zylinderartigen Element mit einem Steuerkopf 120, an dessen Unterseite ein Stababschnitt 130 zentrisch angeordnet ist. Der Stababschnitt 130 besitzt an dem zum Steuerkopf 120 entgegengesetzten Ende einen Fußabschnitt 110. Der Stababschnitt 130 hat einen gleichbleibenden Außendurchmesser. Der Fußabschnitt 110 hat einen Außendurchmesser, der in die zum Steuerkopf 120 entgegengesetzte Richtung zunimmt.

Das Steuerelement 100 ist rotationssymmetrisch aufgebaut und weist einen Innenkanal 160 auf, der sich durch den Kopf 120, den Stababschnitt 130 und den Fußabschnitt 110 konzentrisch erstreckt. Der Innenkanal 160 ist im Kopf 120 derart erweitert, dass sein Innendurchmesser zu zum Fußabschnitt 110 hin weisenden Seite zunimmt, wie dies in Figur 9 gezeigt ist. Somit hat der Innenkanal 160 des Kopfes 120 eine trichterartige Eingangsöffnung, die in Fig. 9 an der linken Seite des Innenkanals 160 dargestellt ist. Die trichterartige Eingangsöffnung des Innenkanals 160 erleichtert es, den Deflectingabschnitt 600 in den Innenkanal 160 einzuführen. Die trichterartige Eingangsöffnung des Innenkanals 160 ist abgerundet, so dass sie keine scharfen Kanten aufweist.

Am Fußabschnitt 110 schwenkt das Steuerelement 100 relativ zum Stabelement 300; daher ist der Fußabschnitt 110 des Steuerelements 100 als Schwenkabschnitt 110 bezeichnet.

Der Schwenkabschnitt 110 besitzt an der zum Steuerkopf 120 entgegengesetzten Seite eine als Fußfläche 110A ausgebildete Endfläche. Im vorliegenden Ausführungsbeispiel ist die Fußfläche 110A nach außen gewölbt, d.h. entgegengesetzt zur die trichterartige Eingangsöffnung des Innenkanals 160 aufweisenden Stirnfläche des Steuerkopfes 120 weg nach außen gewölbt. Anders ausgedrückt nimmt der in Längsrichtung des zylindrischen Steuerelements 100 gemessene Abstand zwischen der Fußfläche 110A zu der zum Fuß entgegengesetzten Stirnfläche des Steuerkopfes 120 vom Außenumfang zur Mitte hin zu. Die Fußfläche 110A bildet daher einen Abschnitt einer Kugelfläche mit einem vorbestimmten Radius, dessen Mittelpunkt auf der gedachten verlängerten Achse des Steuerelements 100 liegt.

An der Fußfläche 110A besitzt der Innenkanal 160 seine Ausgangsöffnung. Der Innenkanal 160 verjüngt sich in dem Bereich des Fußabschnittes 110 und erreicht an der Fußfläche 110A seinen geringsten Innendurchmesser. An der Ausgangsöffnung ist der Innenkanal 160 abgerundet, so dass er keine scharfen Kanten aufweist.

Der Innenkanal 160 hat an dieser engsten Stelle einen Innendurchmesser, der einerseits noch ein sicheres Hindurchgleiten des Deflectingabschnittes 600 und des Katheterschlauches 500 ermöglicht und andererseits eine Führung für den Deflectingabschnitt 600 und den Katheterschlauch 500 bildet, wenn diese hindurchgesteckt sind.

Das Steuerelement 100 ist rotationssymmetrisch, wie dies in den Figuren 9 - 11 erkennbar ist. Das Steuerelement 100 ist aus einem Kunststoffmaterial hergestellt, kann aber auch aus Metall gefertigt sein.

Der Fußfläche 110A des Schwenkabschnittes 110 steht eine Stirnfläche 310A eines Kopfabschnittes 310 des Stabelementes 300 gegenüber, wie dies in den Figuren 9 - 11 dargestellt ist.

Das Stabelement 300 hat einen Längszylinder 320, der an seiner distalen Seite in den Kopfabschnitt 310 übergeht und in der Nähe seines distalen Endabschnitts eine Durchmessererweiterung 330 aufweist. Die Durchmessererweiterung 330 ist als ein Zylinderabschnitt vorgesehen, der einen größeren Außendurchmesser als in dem Abschnitt zwischen dem Kopfabschnitt 310 und der Durchmessererweiterung 330 besitzt. Das Stabelement 300 ist rotationssymmetrisch aufgebaut.

Das Stabelement 300 ist somit ein einstückiges längliches Element, das den Kopfabschnitt 310, den Längszylinder 320 und die Durchmessererweiterung 330 aufweist. Der Längszylinder 320 des Stabelementes 300 mit einer glatten Außenoberfläche ausgebildet.

Das Stabelement 300 ist rotationssymmetrisch aufgebaut und weist einen Innenkanal 350 auf, der sich durch den Kopfabschnitt 310, den Längszylinder 320 und die Durchmessererweiterung 330 konzentrisch erstreckt. Der Innenkanal 350 ist am Kopfabschnitt 310 verengt. Dabei besitzt der Innenkanal 350 an der Stirnfläche 310A des Kopfabschnittes 310 seine Eingangsöffnung, an der der Innenkanal 350 seinen geringsten Innendurchmesser hat. An der Eingangsöffnung ist der Innenkanal 350 abgerundet, so dass er keine scharfen Kanten aufweist. Stromabwärtig der Eingangsöffnung besitzt der Innenkanal 350 einen gleichmäßigen Innendurchmesser, der größer als am Kopfabschnitt 310 ist. Auch an der Ausgangsöffnung ist der Innenkanal 350 abgerundet, so dass er keine scharfen Kanten aufweist. Die Verengung des Innendurchmessers am Kopfabschnitt 310 ermöglicht einerseits noch ein sicheres Hindurchgleiten des Deflectingabschnittes 600 und des Katheterschlauches 500 und bildet andererseits eine Führung für den Deflectingabschnitt 600 und den Katheterschlauch 500, wenn diese hindurchgesteckt sind.

Im vorliegenden Ausführungsbeispiel ist die Stirnfläche 310A ebenfalls nach außen zum Steuerelement 100 hin gewölbt. Anders ausgedrückt erhebt sich die Fußfläche 110A vom Außenumfang zur Mitte hin in Richtung zum Steuerelement 100. Die Stirnfläche 310A bildet dabei einen Abschnitt einer Kugelfläche mit einem vorbestimmten Radius, dessen Mittelpunkt auf der gedachten verlängerten Achse des Stabelements 300 liegt.

Die Fußfläche 110A und die Stirnfläche 310A stehen sich gegenüber und befinden sich in Kontakt zueinander. Somit sitzt der Schwenkabschnitt 110 des Steuerelements 100 mit seiner Fußfläche 110A schwenkbar auf der Stirnfläche 310A des Kopfabschnittes 310 des Stabelements 300. Anders ausgedrückt, kann die Fußfläche 110A auf der Stirnfläche 310A abrollen.

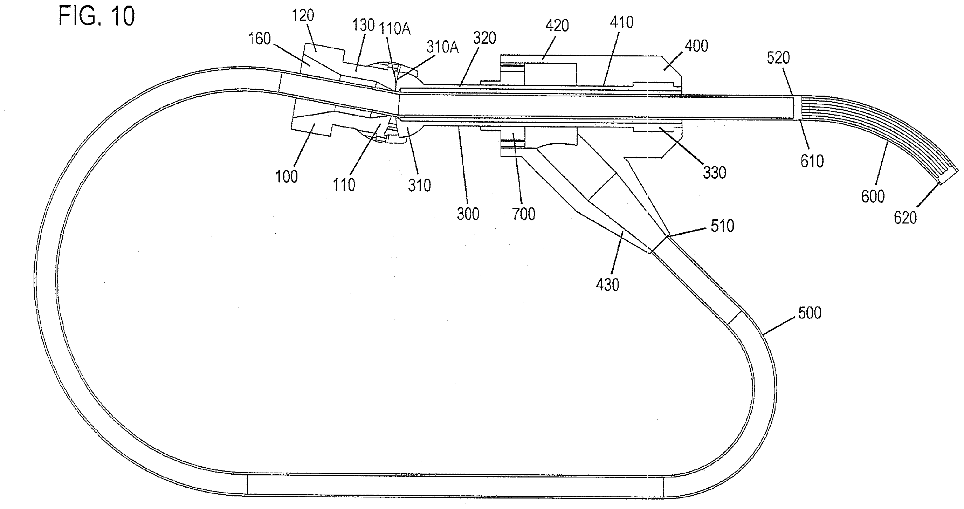

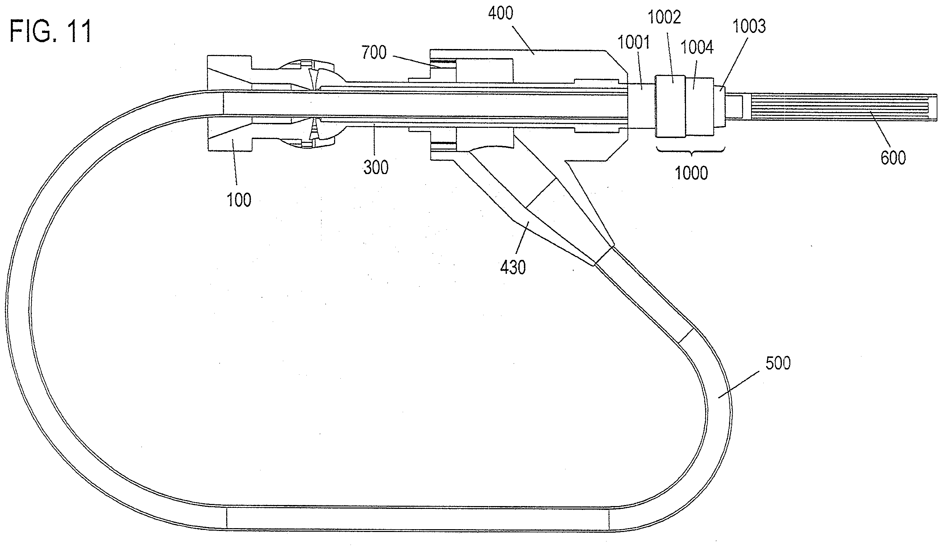

Im nicht geschwenkten Zustand liegen der Schwenkabschnitt 110 des Steuerelements 100 und der Kopfabschnitt 310 des Stabelements 300 auf der gleichen Mittelachse, da im nicht geschwenkten Zustand das Steuerelement 100 und das Stabelement 300 koaxial zueinander angeordnet sind. Somit berühren sich im nicht geschwenkten Zustand die Fußfläche 110A und die Stirnfläche 310A an einer Kreislinie, wie dies in Figur 9 gezeigt ist. Wenn das Steuerelement 100 geschwenkt wird, wie dies in den Figuren 10 und 11 gezeigt ist, also der Schwenkabschnitt 110 relativ zum Kopfabschnitt 310 des Stabelements 300 geneigt wird, rollt die Fußfläche 110A auf der Stirnfläche 310A. Es wird hierbei darauf hingewiesen, dass die Darstellung in den Zeichnungen lediglich schematisch ist.

Das Steuerelement 100 sitzt somit als ein Joystick auf dem Kopfabschnitt 310 des Stabelementes 300.

Das Stabelement 300 ist in dem Stabelementhalter 400 angeordnet. Der Stabelementhalter 400 bildet den Griffkörper des Sekundärendoskops und ist als ein Zylinderelement 420 gebildet, das rotationssymmetrisch aufgebaut ist. Das Zylinderelement 420 weist an der zum Steuerelement 100 hin weisenden Seite einen Hohlraum und einen Boden an der vom Steuerelement 100 abgewandten Seite des Stabelementhalters 400 auf. Der Boden des Stabelementhalters 400 weist einen konzentrischen Innenkanal 410 auf. In dem konzentrischen Innenkanal 410 ist an einem Abschnitt von diesem ein Hohlraum ausgearbeitet, in welchem die Durchmessererweiterung 330 des Stabelementes 300 sitzt. Die axialen Endflächen des Hohlraums in der Nähe des Bodens des Stabelementhalters 400 bilden jeweilige Anschläge für die axialen Endflächen der Durchmessererweiterung 330. In diesem Ausführungsbeispiel kann sich somit das Stabelement 300 ist axial relativ zum Stabelementhalter 400 nicht oder lediglich mit einem geringfügigen Spiel bewegen.

An einem Abschnitt seiner Außenumfangsseite besitzt das Zylinderelement 420 des Stabelementhalters 400 ein Katheteranschlusselement 430. Im vorliegenden Ausführungsbeispiel erstreckt sich das Katheteranschlusselement 430 unter einem spitzen Winkel relativ zu dem Zylinderelement 420 des Stabelementes 400, wie dies aus den Zeichnungen hervorgeht.

Insbesondere ist das Katheteranschlusselement 430 als ein rundes Hohlprofil ausgearbeitet, das quasi eine Kanalabzweigung aus dem distalen Hohlraum des Zylinderelementes 420 darstellt. Das Katheteranschlusselement 430 ist zylinderartig ausgebildet, wobei es sich in Richtung vom Zylinderelement 420 weg verjüngt. Im Inneren besitzt das Katheteranschlusselement 430 einen konzentrischen Kanal, in welchem die Drahtkörper geführt werden. An seinem distalen Ende besitzt das Katheteranschlusselement 430 eine kreisartige Mündung.

An der kreisartigen Mündung des Katheteranschlusselementes 430 ist der Katheterschlauch 500 befestigt. Insbesondere sitzt das proximale Ende 510 des Katheterschlauches 500 an der Mündung des Katheteranschlusselementes 430.

An seinem distalen Ende besitzt der Katheterschlauch einen in ihm aufgenommenen Ring 520. Der Ring 520 bildet das distale Ende des Katheterschlauches und den Übergang zu dem Deflectingabschnitt 600.

Der Katheterschlauch 500 und der Deflectingabschnitt 600 sind ähnlich aufgebaut wie der Katheterschlauch 5 und der Deflectingabschnitt 6 des ersten Ausführungsbeispiels.

Ähnlich wie in Fig. 4 sind auch im zweiten Ausführungsbeispiel die Drahtelemente an Einhängausbauchungen des Steuerelements eingehängt. Die Beschreibung in Zusammenhang mit den Drahtelementen des ersten Ausführungsbeispiels gilt auch im zweiten Ausführungsbeispiel und wird nicht wiederholt. Der Aufbau, die Führung und Funktionsweise der Drahtelemente ist gleich.

Lediglich der Drahtführungsring 700 hat im zweiten Ausführungsbeispiel zusätzlich zu seinem im ersten Ausführungsbeispiel beschriebenen Aufbau eine weitere Funktion. Der Drahtführungsring 700 als solcher ist wie im ersten Ausführungsbeispiel aufgebaut. Der Drahtführungsring 700 des zweiten Ausführungsbeispiels ist längs zum Zylinderelement 420 des Stabelementhalters 400 verschiebbar. Zu diesem Zweck weist das Zylinderelement 420 des Stabelementhalters 400 einen in den Zeichnungen nicht gezeigten axialen Längsschlitz auf, entlang welcher die Befestigungsschraube, die in die Gewindebohrung 72 (Figur 4) eingreift und den Drahtführungsring 700 am Zylinderelement 420 des Stabelementhalters 400 fixieren kann, an verschiedenen Positionen festgeschraubt werden kann.

Die Drähte können hierbei gespannt werden, indem das Ringelement 700 in axialer Richtung in den Hohlraum des Zylinderelements 420 des Stabelementhalters 400 hinein verschoben und dann per Feststellelement arretiert wird. Dadurch ändert sich der Winkel des Ablenkungspunktes der Drähte am Ausgang des Ringelementes 700; das heißt an der rechten Seite des Ringelementes 700 in Fig. 9. Anders ausgedrückt wird beim Spannen der Drähte der stumpfe Winkel des Ablenkungspunktes der Drähte am stromabwärtigen Ausgang des Ringelementes 700 zwischen der Erstreckung der Drähte vom Einhängpunkt zum Ringelement 700 und der Erstreckung der Drähte vom Ringelement 700 zum Katheterschlauch 500 verkleinert. Dadurch können kleine Spannlängen der Drähte verwirklicht werden.

Die Drahtkörper sind durch den Katheterschlauch 500 und durch den Ring 520 des Katheterschlauches hindurchgeführt und sind an der Deflectingkappe 620 des Deflectingabschnittes 600 verankert. Insbesondere sind die Drahtkörper so an der Deflectingkappe 620 verankert, dass sie gleichmäßig zueinander beabstandet und in der gleichen Reihenfolge wie am Schwenkabschnitt 110 angeordnet sind.

Der Ring 520 weist Öffnungen für die Drahtkörper in entsprechender Weise wie bei der Ausgestaltung des Drahtführungsringes 700 auf.

Die Länge jedes Drahtkörpers vom Befestigungspunkt an der Deflectingkappe 620 bis zum Befestigungspunkt am Schwenkabschnitt 110 ist stets gleich.

### Funktionsweise

Ähnlich wie im ersten Ausführungsbeispiel kann auch das Steuerelement 100 des zweiten Ausführungsbeispiels wie ein Joystick betätigt werden, wobei die Fußfläche 110A des Hohlkugelabschnitts 110 auf der Stirnfläche 310A des Kopfabschnittes 310 abrollt. Ein Schwenkvorgang des Joysticks 100 relativ zum Stabelement 300 in beliebiger Richtung ist dadurch möglich. Die Richtung und das Ausmaß der Auslenkbewegung des Joysticks 100 relativ zum Stabelement 300 wird dann durch die an der Deflectingkappe 620 angeordneten Drahtkörper auf den als biegbaren Körper ausgestalteten Deflectingabschnitt 600 übertragen.

Wenn der Joystick 100 relativ zum Stabelement 300 nach links bewegt wird, vollführt der Deflectingabschnitt 600 eine nach links gerichtete Bewegung, wie dies in Figur 10 gezeigt ist. Wenn der Joystick 100 relativ zum Stabelement 300 nach rechts bewegt wird, vollführt der Deflectingabschnitt 600 eine nach rechts gerichtete Bewegung, wie dies in Figur 11 gezeigt ist.

Das Steuerelement 100 wird vor dem Einführen des Deflectingabschnitts 600 gerade gestellt, so dass der Deflectingabschnitt 600 und der am Deflectingabschnitt 600 angrenzende Abschnitt des Katheterschlauches 500 gerade ausgerichtet sind. Das distale Ende (an der Deflectingkappe 620) des Deflectingabschnitts 600 wird in die trichterartige Eingangsöffnung des Innenkanals 160 im Steuerelement 100 eingeführt, durch den Innenkanal 160 geschoben, in die zur Ausgangsöffnung des Innenkanals 160 benachbarten Eingangsöffnung des Innenkanals 350 im Stabelement 300 eingeführt und durch den Innenkanal 350 hindurch geschoben, bis der Deflectingabschnitt 600 an der zu der trichterartigen Eingangsöffnung des Innenkanals 350 entgegengesetzten Ausgangsöffnung des Innenkanals 350 heraustritt.

Wenn der Deflectingabschnitt 600 seine beabsichtigte durch das Sekundärendoskop hindurchgeschobene Betriebsstellung erreicht hat, kann durch in der erwünschten Richtung und um das erwünschte Maß erfolgendes Schwenken des Steuerelementes 100 der Deflectingabschnitt 600 in die erwünschte Stellung gebracht werden.

### Alternativen des Sekundärendoskops

Der Drahtkörper 2 ist in der in Figur 4 gezeigten Einhängausbauchung 14 in der Form eines Tonnennippels 21 eingehängt. Die Erfindung ist nicht auf ein Tonnennippel beschränkt und das Nippel 21 kann als ein bekanntes Birnennippel ausgeführt sein, beliebige ähnliche Nippel können angewendet werden. Die Form der Einhängausbauchung 14 kann an die gewählte Nippelform angepasst werden.

Im ersten Ausführungsbeispiel ist die Größe des Hohlkugelabschnittes 11 so gewählt worden, dass sie 9/10 einer Kugel beträgt. Die Erfindung ist nicht darauf beschränkt. Eine beliebige Hohlkugelformgröße des Hohlkugelabschnittes 11 kann gewählt werden, solange diese noch an dem Gegenkugelabschnitt 31 die Schwenkbewegung ausführen kann. Der Hohlkugelabschnitt 11 kann auch eine Hohlkugelringabschnittform haben, die sich um ein vorbestimmtes Mindestmaß parallel zur Achsrichtung des Steuerelements 1 zu beiden Seiten von der Äquatorlinie erstreckt und quasi ein Äquatorband bildet.

Im zweiten Ausführungsbeispiel ist die Fußfläche 110A nach außen gewölbt. Außerdem ist die Stirnfläche 310A des Kopfabschnittes 310 nach außen gewölbt. Die Erfindung ist nicht darauf beschränkt. Das Sekundärendoskop kann unter Ausnutzung des Prinzips der Erfindung auch so aufgebaut sein, dass die Fußfläche 110A eben gestaltet ist und die Stirnfläche 310A nach außen gewölbt ist. Andererseits kann das Sekundärendoskop auch so aufgebaut sein, dass die Fußfläche 110A nach außen gewölbt ist und die Stirnfläche 310A eben gestaltet ist. Es ist auch eine Konstruktion denkbar, bei der die Stirnfläche 310A nach innen gewölbt ist und die Fußfläche 110A nach außen gewölbt ist, sofern der Krümmungsradius der Stirnfläche 310A größer als der Krümmungsradius der Fußfläche 110A ist. In ähnlicher Weise kann die Stirnfläche 310A nach außen gewölbt sein und die Fußfläche 110A nach innen gewölbt sein, sofern der Krümmungsradius der Stirnfläche 310A kleiner als der Krümmungsradius der Fußfläche 110A ist. Es ist lediglich ausreichend, dass die Fußfläche 110A sicher und kontrolliert auf der Stirnfläche 310A abrollen kann.

Im ersten Ausführungsbeispiel dient das Vierkantende 34 der Ermöglichung einer Schraubbewegung, um an den Gewindeabschnitten 33 und 41 eine Relativbewegung des Stabelementes 3 zum Stabelementhalter 4 auszuführen, um die Drahtkörper 2 zu spannen. Die Erfindung ist nicht auf die Vierkantform am Ende 34 des Stabelementes 3 beschränkt. Eine Dreikantform, Achtkantform, andere polygonale Form kann gewählt werden. Im Prinzip kann jede beliebige Form, die das Angreifen eines die Drehbewegung des Stabelementes am Ende 34 erzeugenden Momentes ermöglicht, gewählt werden.

Im ersten Ausführungsbeispiel wird die Relativbewegung des Stabelementes 3 zum Stabelementhalter 4 durch die Gewindeabschnitte 33 und 41 bewerkstelligt. Durch die Bewegung des Stabelementes 3 relativ zum Stabelementhalter 4 werden die Drahtkörper 2 gespannt. Eine beliebige andere Bewegungsart des Stabelementes 3 zum Stabelementhalter 4 kann für diesen Zweck gewählt werden. Beispielsweise kann der Stabelementhalter 4 ein durchgehendes Innenzylinderloch aufweisen und das Stabelement 3 einen durchgehend zylindrischen Längszylinder 32 haben, wobei am Ende 34 des Stabelementes 3 eine Zugeinrichtung angebracht ist. Im Stabelementhalter 4 kann eine Gewindebohrung senkrecht zur Achse des Stabelementhalters vorgesehen werden, in der eine Feststellschraube sitzt, die den Längszylinder 32 in beliebiger Stellung relativ zum Stabelementhalter 4 arretieren kann.

Im ersten Ausführungsbeispiel ist das Stabelement 3 relativ zum Stabelementhalter 4 durch eine Schraubbewegung konzentrisch hinein oder herausschraubbar, wobei bei der Schraubbewegung das Innengewinde 41 des Stabelementhalters 4 am Außengewinde 33 des Stabelementes 3 in Gewindeeingriff stehen. Bei dieser Ausführung sind die Drähte 2 spannbar, indem das Stabelement 3 relativ zum Stabelementhalter 4 bewegt und arretiert wird.

Im zweiten Ausführungsbeispiel sitzt das Stabelement 300 im Stabelementhalter 400, in dem die Durchmessererweiterung 330 in einem für diese vorgesehenen Hohlraum des Stabelementhalters 400 angeordnet ist. Die distale und proximale Endfläche der Durchmessererweiterung 330 bildet dabei jeweils einen Anschlag am Hohlraum des Stabelementhalters 400. Bei dieser Ausführung sind die Drähte spannbar, indem das Ringelement 700 in axialer Richtung in den Hohlraum des Zylinderelements 420 des Stabelementhalters 400 hinein verschoben und dann per Feststellelement arretiert wird.

Die Erfindung ist nicht darauf beschränkt. Im ersten Ausführungsbeispiel kann die für das zweite Ausführungsbeispiel vorgesehene Drahtspannmöglichkeit angewendet werden; und im zweiten Ausführungsbeispiel kann die für das erste Ausführungsbeispiel vorgesehene Drahtspannmöglichkeit angewendet werden.

In den Ausführungsbeispielen erstreckt sich das Katheteranschlusselement 43; 430 unter einem spitzen Winkel unter Betrachtung der Figuren zum Stabelementhalter 4; 400. Die Erfindung ist nicht darauf beschränkt. Ein beliebiger Erstreckungswinkel des Katheteranschlusselementes zum Stabelementhalter kann gewählt werden.

In den Ausführungsbeispielen ist der Katheterschlauch 5; 500 ein Übertragungsführungskörper, der einen Hohlraum besitzt, in dem der Bewegungsübertragungsdrahtkörper geführt wird. Bei einer Schwenkbewegung des Steuerelementes sind die Bewegungsübertragungsdrahtkörper 2 Zugkräften und Druckkräften ausgesetzt. Wenn diese Zugkräfte und Druckkräfte auf sie ausgeübt werden, müssen die Bewegungsübertragungsdrahtkörper 2 am Übertragungsführungskörper gleiten können. Der Übertragungsführungskörper kann hierbei einen geschlossenen Querschnitt haben, wie dies bei einem Katheterschlauch 5; 500 der Fall ist. Die Erfindung ist nicht darauf beschränkt. Der Übertragungsführungskörper kann ein Schienenelement oder Kastenelement sein, an dem der Bewegungsübertragungsdrahtkörper geführt wird. Der Querschnitt des Übertragungsführungskörpers kann an der Seite offen sein, an der die Bewegungsübertragungsdrahtkörper 2 nicht gleiten.

Das Steuerelement 1; 100 kann arretierbar sein, um eine Auslenkstellung des Steuerelements 1; 100 zu arretieren. Im ersten Ausführungsbeispiel kann die Arretierung durch eine Feststellschraube erfolgen, die z.B. den Hohlkugelabschnitt 11 durchdringt und an der Oberfläche des Gegenkugelabschnittes 31 angreift und somit als eine Reibungsbremse so wirkt, dass eine bestimmte Auslenkstellung des Steuerelements 1 also des Steuerhebels per Reibungsbremse arretierbar ist. Darüber hinaus kann in allen Ausführungsbeispielen eine Arretierung erfolgen, indem der/die Bewegungsübertragungsdrahtkörper 2 z.B. am Drahtführungsring 7; 700 oder am Zylinderelement 42; 420 des Stabelementhalters 4; 400 geklemmt werden. Sollten alle Drähte 2 z.B. durch eine am Drahtführungsring 7; 700 oder am Zylinderelement 42; 420 angebrachte Feststellklemme arretiert werden, wird dadurch eine sichere Arretierung einer Auslenkstellung des Steuerelements 1; 100 erreicht. Andere technische Möglichkeiten zum Blockieren der Drähte 2 können gewählt werden.

### Montage am Mutterendoskop

Figur 11 zeigt eine schematische Schnittdarstellung des Sekundärendoskops des zweiten Ausführungsbeispiels, wobei an der distalen Seite des Griffkörpers ein Montagekörper zur Montage am Mutterendoskop sitzt.

Wie dies in Figur 11 gezeigt ist, sitzt am distalen Ende des als Katheterkanals fungierenden Innenkanals ein Anschlussstutzen 1001. Der Anschlussstutzen 1001 ist ein Rohrelement. Das Rohrelement verlängert den Innenkanal. Das proximale Ende des Anschlussstutzens 1001 ist mit dem distalen Ende des den Griffkörper des Sekundärendoskops bildenden Stabelementhalters 4 drehfest verbunden. Das distale Ende des Anschlussstutzens 1001 ist mit einem proximalen Ende eines Montagekörpers 1000 des Sekundärendoskops drehfest verbunden. Der Montagekörper 1000 ist ebenfalls hohl und verlängert den Innenkanal.

Der Montagekörper 1000 des Sekundärendoskops dient der Montage des Sekundärendoskops am Mutterendoskop und bildet mit einem nachstehend beschriebenen Montagekörper 2440 des Mutterendoskops eine Montageeinheit.

Im vorliegenden Ausführungsbeispiel ist der Montagekörper 1000 als Luer-Lock-Maleelement ausgebildet. Die Montageeinheit wird durch eine Luer-Lock-Verbindung mit einem axial verlaufenden Innenkanal gebildet.

Das Luer-Lock-Maleelement 1000 ist ein Element dieser Luer-Lock-Verbindung. Luer-Lock-Verbindungen sind genormte Verbindungen, die im medizinischen Bereich häufig eingesetzt werden.

Insbesondere hat das hohle Luer-Lock-Maleelement 1000 an der proximalen Seite ein hohles Griffelement 1002 und an der distalen Seite einen hohlen Luer-Lock-Konus 1003, von dem der proximale Abschnitt durch eine Luer-Lock-Hülse 1004 umgegeben ist. Das proximale Ende der Luer-Lock-Hülse 1004 liegt am distalen Ende des Griffelementes 1002 an und ist mit diesem verbunden. Da der genaue Aufbau einer Luer-Lock-Verbindung der Fachwelt bekannt ist, wird dieser hier nicht genauer erläutert.

Das Griffelement 1002 kann an seiner Außenumfangsfläche mit einer Riffelung versehen sein. Das Griffelement 1002 kann alternativ oder zusätzlich an seiner Außenumfangsfläche mit einer Markierung versehen sein, die über den montierten oder demontierten Zustand informiert. D.h. wenn die Luer-Lock-Verbindung geschlossen ist, wird dies dem Anwender durch eine Markierung angezeigt; und wenn die Luer-Lock-Verbindung geöffnet ist, wird dies dem Anwender durch eine Markierung ebenfalls angezeigt. Eine solche anzeigende Markierung kann zum Beispiel ein ein- und ausfahrenbarer Vorsprung sein, der z.B. in eingefahrener Stellung eine geschlossene Verbindung anzeigt und in ausgefahrener Stellung eine offene Verbindung anzeigt. Dadurch wird für den Anwender die Information, dass die Verbindung nicht sicher verbunden ist, auch durch Tasten erkennbar. Der ein- und ausfahrenbare Vorsprung kann über einen rot gefärbten Umfangsrand verfügen, der im eingefahrenen Zustand versenkt ist und im ausgefahrenen Zustand für den Anwender sichtbar ist und über die offene Verbindung informiert.

Alternativ kann der Montagekörper 1000 ein Schraubverschlusselement, ein Bajonettverschlusselement oder ein Einrastverbindungselement sein und dementsprechend mit dem Montagekörper 2440 des Mutterendoskops eine lösbare Schraubverbindung, Bajonettverschlussverbindung oder Einrastverbindung bilden. In jedem Fall verfügt diese lösbare Montageverbindung über einen Innenkanal und über eine Arretierung. Die Arretierung ist vorzugsweise durch den Anwender nicht lösbar, und kann z.B. vom Gerätehersteller oder in einem dazu befugten Wartungs- und Reparaturzentrum geöffnet werden.

### Kombination aus Mutterendoskop und Sekundärendoskop

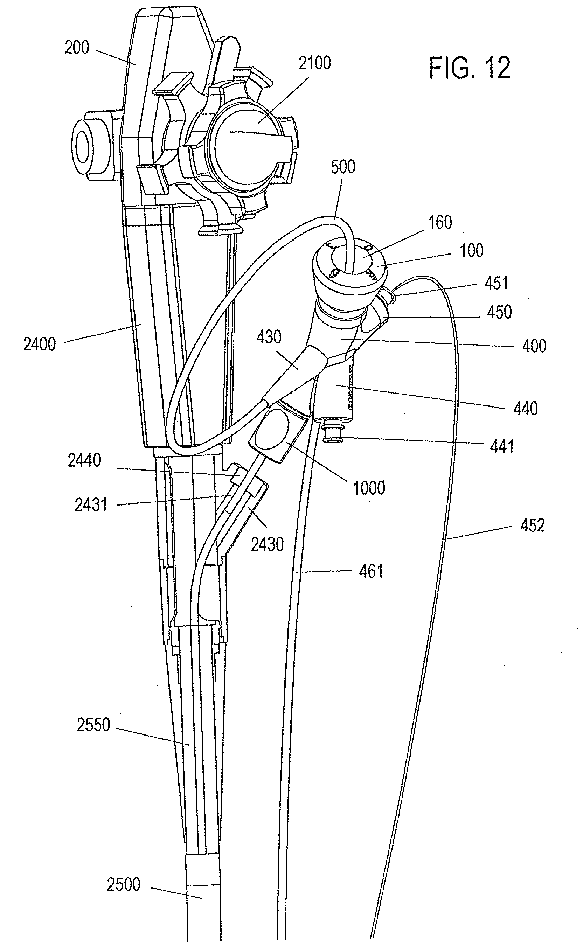

Figur 12 zeigt eine schematische Darstellung im Teilschnitt einer Kombination aus einem Mutterendoskop und dem erfindungsgemäßen Sekundärendoskop.

Ein Mutterendoskop 200 hat ein Steuerelement 2100 an einem Mutterendoskopgriff 2400. An der distalen Seite des Mutterendoskopgriffes 2400 erstreckt sich unter einem vorbestimmten Winkel, der 45° betragen kann, ein Zugangsanschluss 2430 für ein Sekundärendoskop. Der vorbestimmte Winkel ist nicht auf 45° beschränkt.

Ein Mutterendoskop 200 besitzt einen zentrisch vorgesehen Arbeitskanal 2550, der im Schlauchelement 2500 des Mutterendoskops weiterläuft. Vom Arbeitskanal 2550 zweigt eine Arbeitskanalabzweigung 2431 in den Zugangsanschluss 2430 ab. Im Zugangsanschluss 2430 verläuft die Arbeitskanalabzweigung 2431 ebenfalls zentrisch. Der Ausgang der Arbeitskanalabzweigung 2431 mündet somit in den Arbeitskanal 2550 des Mutterendoskops. Der Eingang der Arbeitskanalabzweigung 2431 besitzt eine Öffnung an der proximalen Seite des Zugangsanschlusses 2430. An dieser Öffnung ist ein Luer-Lock-Femaleelement 2440 als Montagekörper für die Verbindung mit dem Sekundärendoskop drehsicher angebracht. Das Luer-Lock-Femaleelement 2440 wird auch als Hülse bezeichnet.

Am Luer-Lock-Femaleelement 2440 wird ein erfindungsgemäßes Sekundärendoskop montiert. Dabei wird der Montagekörper 1000 des Sekundärendoskops mit dem Montagekörper 2440 des Mutterendoskops verbunden und arretiert.

Das Sekundärendoskop aus Fig. 12 ist ein erfindungsgemäßes Sekundärendoskop gemäß den vorstehend beschriebenen Ausführungsbeispielen, das mit weiteren Funktionseinheiten versehen sein kann, wie dies nachstehend beschrieben ist. Das Sekundärendoskop besitzt den Griffkörper 400, in welchem der Katheterkanal 410 ausgebildet ist. Wie dies in Fig. 12 gezeigt ist, hat der Griffkörper 400 an der proximalen Seite das Steuerelement 160. Ebenfalls an der proximalen Seite des Griffkörpers 400 ist ein Zusatzkanalanschlusselement 450 angeordnet, das sich winklig vom Griffkörper 400 weg z.B. in proximaler Richtung erstreckt. Im Zusatzkanalanschlusselement 450 erstreckt sich ein Zusatzkanal in den Griffkörper 400 und mündet in den Innenkanal/Katheterkanal. In den Zusatzkanal können weitere medizinische Instrumente eingeführt werden. Ein Beispiel für ein solches Instrument ist ein Laserkabel 452.

An der proximalen Seite des Zusatzkanalanschlusselementes 450 ist eine Zusatzkanaleingangsöffnung 451 angeordnet, in die das Laserkabel 452 eingeführt ist. An der distalen Seite hat der Griffkörper 400 den Montagekörper 1000, wie bereits beschrieben wurde. Ebenfalls an der distalen Seite des Griffkörpers 400 ist ein Spülkanalanschlusselement 440 angeordnet, das sich winklig vom Griffkörper 400 weg z.B. in distaler Richtung erstreckt. An der distalen Seite des Spülkanalanschlusselementes 440 befindet sich eine Spülkanaleingangsöffnung 441. An der distalen Seite hat der Griffkörper 400 außerdem ein Stromversorgungskabel 461.

Wie bereits erläutert, hat der Griffkörper 400 außerdem an der distalen Seite das Katheteranschlusselement 430, von dem sich distal der Katheterschlauch 500 erstreckt. Der Katheterschlauch 500 ist in die Eingangsöffnung des Katheterkanals 160 des Steuerelementes 100 eingeführt und erstreckt sich durch den in Fig. 12 nicht gezeigten Katheterkanal 410, durch den Innenkanal des Montagekörpers 1000 des Sekundärendoskops, durch den Innenkanal des Montagekörpers 2440 des Mutterendoskops, durch die Arbeitskanalabzweigung 2431 und durch den Arbeitskanal 2550 des Mutterendoskops bis hin zu seinem Deflectingabschnitt 600. Durch Schwenkbewegung des Steuerelements 100 wird der Deflectingabschnitt 600 geschwenkt.

Am Steuerelement 100 können Markierungen vorgesehen sein, die dem Anwender die Schwenkrichtungen für den Deflectingabschnitt 600 anzeigen. Zum Beispiel kann, indem das Steuerelement 100 nach rechts, d.h. in die Richtung, die durch die Markierung "R" angegeben ist, geschwenkt wird, der Deflectingabschnitt 600 nach rechts geschwenkt werden. Indem das Steuerelement 100 nach links, d.h. in die Richtung, die durch die Markierung "L" angegeben ist, geschwenkt wird, kann der Deflectingabschnitt 600 nach links geschwenkt werden. Indem das Steuerelement 100 in die anderen durch Markierungen "D" oder "U" geschwenkt wird, wird der Deflectingabschnitt 600 entsprechend nach unten oder nach oben geschwenkt. Da das Steuerelement 100 in alle Richtungen geschwenkt werden kann, kann auch der Deflectingabschnitt 600 entsprechend in alle Richtungen geschwenkt werden.

### Vorteile der Erfindung

Durch die spezifische Anordnung des Katheterschlauches 500 im Katheterkanal 160, 410 des Sekundärendoskopgriffkörpers 400 bildet der Katheter quasi in sich selbst eine Schleife. Darüber hinaus kann der Katheterkanal 160, 410 im Sekundärendoskop außerordentlich kurz gestaltet werden, was zu einem äußerst kleinen Aufbau des Sekundärendoskops als ganzes führt. Dadurch wird für den Anwender die Bedienung einfacher und übersichtlicher, da der Katheterschlauch 500 kein Hindernis für den Anwender bildet. Die Bedienbarkeit des Steuerelements als Joystick erleichtert die Handhabung. Das Sekundärendoskop ist durch eine Hand betätigbar. Darüber hinaus kann das Sekundärendoskop aufgrund seines einfachen Aufbaus kostengünstig hergestellt werden.

### Bezugszeichenliste

1 Steuerelement; Joystick
2, 2a, 2b Drahtkörper
3 Basiselement; Stabelement
4 Stabelementhalter; Griffkörper des Sekundärendoskops
5 Katheterschlauch
6 Deflectingabschnitt
7 Drahtführungsring
11 Hohlkugelabschnitt
11A Fußfläche des Hohlkugelabschnitts 11
12 Kopf des Steuerelementes 1
13 Stababschnitt
14 Einhängausbauchung
15 Drahtkörpereinhängung
16 Innenkanal im Steuerelement; Katheterkanal
21 Tonnennippel
31 Kopfabschnitt; Gegenkugelabschnitt
31A Stirnfläche des Kopfabschnittes 31
32 Längszylinder
33 Gewindeabschnitt des Stabelementes 3
34 Vierkantende; distales Ende des Stabelementes 3
35 Innenkanal im Stabelement; Katheterkanal
41 Gewindeabschnitt des Stabelementhalters 4
42 Zylinderelement
43 Katheteranschlusselement
51 Katheterschlauchanschluss
52 Ring
61 Deflectinganschluss
62 Deflectingkappe
71 Drahtführungsbohrung
72 Gewindebohrung für Befestigungsschraube
100 Steuerelement; Joystick
110 Hohlkugelabschnitt
110A Fußfläche des Hohlkugelabschnitts 110
120 Kopf des Steuerelementes 100
130 Stababschnitt
160 Innenkanal im Steuerelement; Katheterkanal
200 Mutterendoskop
300 Basiselement; Stabelement
310 Kopfabschnitt; Gegenkugelabschnitt
310A Stirnfläche des Kopfabschnittes 31
320 Längszylinder
330 Durchmessererweiterung
350 Innenkanal im Stabelement; Katheterkanal
400 Stabelementhalter; Griffkörper des Sekundärendoskops
410 Innenkanal im Stabelement
420 Zylinderelement
430 Katheteranschlusselement
440 Spülkanalanschlusselement
441 Spülkanaleingangsöffnung
450 Zusatzkanalanschlusselement
451 Zusatzkanaleingangsöffnung
452 Laserkabel
461 Stromversorgungskabel
500 Katheterschlauch
510 Katheterschlauchanschluss
520 Ring
600 Deflectingabschnitt
610 Deflectinganschluss
620 Deflectingkappe
700 Drahtführungsring
1000 Luer-Lock-Maleelement; Montagekörper
1001 Anschlussstutzen
1002 Griffelement
1003 Luer-Lock-Konus
1004 Luer-Lock-Hülse
2100 Steuerelement
2400 Mutterendoskopgriff
2430 Zugangsanschluss für ein Sekundärendoskop
2431 Arbeitskanalabzweigung im Zugangsanschluss
2440 Luer-Lock-Femaleelement; Montagekörper
2500 Schlauchelement des Mutterendoskops
2550 Arbeitskanal im Mutterendoskop

## Patentansprüche

1. An ein Mutterendoskop anbringbares Sekundärendoskop mit einem Griffkörper (4; 400), einem am Griffkörper (4; 400) angebrachten Katheterschlauch (5; 500);
einem schwenkbaren Steuerelement (1; 100) an der proximalen Seite des Griffkörpers (4; 400) zur Steuerung eines am distalen Ende des Katheterschlauchs (5; 500) angeordneten Biegeabschnittes (6; 600), und einem Montageanschluss an der distalen Seite des Griffkörpers (4; 400) zum Befestigen des Sekundärendoskops an einem Mutterendoskop (200), wobei der Montageanschluss am Außenkörper des Griffkörpers (4; 400) angebracht, **gekennzeichnet dadurch, dass**
der Griffkörper (4;400) einen Außenkörper mit einem Innenkanal und einen im Innenkanal des Außenkörpers angeordneten Innenkörper (3; 300) aufweist, wobei der Innenkörper (3; 300) zum Außenkörper bewegbar ist, und dass
das Steuerelement (1; 100) an der proximalen Seite des Innenkörpers (3; 300) angeordnet ist und relativ zum Innenkörper (3; 300) schwenkbar ist.

2. Sekundärendoskop gemäß Anspruch 1, wobei
der Griffkörper (4; 400) einen Katheterkanal (16, 35; 160) zur Führung des Katheterschlauchs (5; 500) aufweist.

3. Sekundärendoskop gemäß Anspruch 2, wobei
das proximale Ende des Steuerelementes (1; 100) eine proximale Eingangsöffnung des Katheterkanals (16, 35; 160) aufweist.

4. Sekundärendoskop gemäß einem der Ansprüche 1 bis 3, wobei
das proximale Ende des Katheterschlauchs (5; 500) an der distalen Seite des Griffkörpers (4; 400) angebracht ist.

5. Sekundärendoskop gemäß einem der Ansprüche 1 bis 4, wobei
das proximale Ende des Katheterschlauchs (5; 500) an der Außenseite des Griffkörpers (4; 400) angebracht ist.

6. Sekundärendoskop gemäß einem der Ansprüche 1 bis 5, wobei
das distale Ende des Griffkörpers (4; 400) einen Montagekörper (1000) zur Montage am Mutterendoskop aufweist, wobei der Montagekörper (1000) einen Abschnitt des Katheterkanals (16, 35; 160) zur Führung des Katheterschlauchs (5; 500) aufweist.

7. Sekundärendoskop gemäß Anspruch 6, wobei
der Montagekörper (1000) mit einer Markierung versehen ist, die über den montierten oder demontierten Zustand informiert.

8. Sekundärendoskop gemäß Anspruch 6 oder 7, wobei
der Montagekörper (1000) ein Luer-lock-Verbindungselement, ein Schraubverschlusselement, ein Bajonettverschlusselement oder ein Einrastverbindungselement ist.

9. Sekundärendoskop gemäß einem der Ansprüche 1 bis 8, wobei
das Steuerelement (1; 100) zum Schwenken des Biegeabschnittes (6; 600) schwenkbar ist und an seiner proximalen Seite mit Markierungen versehen ist, die über die Schwenkrichtung des Biegeabschnittes (6; 600) informieren.

10. Sekundärendoskop gemäß einem der Ansprüche 1 bis 9, wobei
der Katheterschlauch (5; 500) am Außenkörper des Griffkörpers (4; 400) angebracht ist.

11. Kombination aus Mutterendoskop und Sekundärendoskop, mit
einem Mutterendoskop (200) mit einem Zugangsanschluss (2430) für ein Sekundärendoskop; und
einem Sekundärendoskop gemäß einem der Ansprüche 1 bis 10.

12. Kombination aus Mutterendoskop und Sekundärendoskop, mit
einem Mutterendoskop (200) mit einem Zugangsanschluss (2430) für ein Sekundärendoskop an der distalen Seite eines Mutterendoskopgriffes (2400); und
einem am Zugangsanschluss (2430) lösbar montierten Sekundärendoskop gemäß einem der Ansprüche 1 bis 10 mit einem Sekundärendoskopgriff (4; 400) und einem Katheterschlauch (5; 500);
wobei der Katheter (5; 500) des Sekundärendoskops durch einen Katheterkanal (16; 160, 410) des Sekundärendoskopgriffs (4; 400) geführt ist;
wobei das distale Ende des Sekundärendoskopgriffs (4; 400) am Zugangsanschluss (2430) des Mutterendoskops (200) lösbar montiert ist;
wobei in der montierten Position das Sekundärendoskop am Mutterendoskopgriff (2400) an der proximalen Seite des Zugangsanschlusses (2430) des Mutterendoskops lösbar montiert ist;
wobei am Sekundärendoskop eine Kathetereingangsöffnung proximal vom Montageort des Sekundärendoskops positioniert ist.

13. Kombination gemäß Anspruch 12, wobei
der Sekundärendoskopgriff (4; 400) des Weiteren eine Zusatzkanaleingangsöffnung (451) an der proximalen Seite, und eine Fluideingangsöffnung an der distalen Seite aufweist.

## Claims

1. Secondary endoscope which can be attached to a parent endoscope, comprising
a handle body (4; 400),
a catheter tube (5; 500) attached to the handle body (4; 400);
a pivotable control element (1; 100) on the proximal side of the handle body (4; 400) for controlling a bending portion (6; 600) arranged on the distal end of the catheter tube (5; 500),
and
a mounting connection on the distal side of the handle body (4; 400) for fastening the secondary endoscope to a parent endoscope (200), wherein the mounting connection is attached to the outer body of the handle body (4; 400),
**characterized in that**
the handle body (4; 400) has an outer body with an inner duct and an inner body (3; 300) arranged in the inner duct of the outer body, wherein the inner body (3; 300) is movable with respect to the outer body, and **in that**
the control element (1; 100) is arranged on the proximal side of the inner body (3; 300) and is pivotable relative to the inner body (3; 300).

2. Secondary endoscope according to Claim 1, wherein the handle body (4; 400) has a catheter duct (16, 35; 160) for guiding the catheter tube (5; 500).

3. Secondary endoscope according to Claim 2, wherein the proximal end of the control element (1; 100) has a proximal inlet opening of the catheter duct (16, 35; 160).

4. Secondary endoscope according to one of Claims 1 to 3, wherein
the proximal end of the catheter tube (5; 500) is attached to the distal side of the handle body (4; 400).

5. Secondary endoscope according to one of Claims 1 to 4, wherein
the proximal end of the catheter tube (5; 500) is attached to the outer side of the handle body (4; 400).

6. Secondary endoscope according to one of Claims 1 to 5, wherein
the distal end of the handle body (4; 400) has a mounting body (1000) for mounting on the parent endoscope, wherein the mounting body (1000) has a portion of the catheter duct (16, 35; 160) for guiding the catheter tube (5; 500).

7. Secondary endoscope according to Claim 6, wherein the mounting body (1000) is provided with a marking which informs about the mounted or demounted state.

8. Secondary endoscope according to Claim 6 or 7, wherein
the mounting body (1000) is a Luer lock connection element, a screw fastener element, a bayonet fastener element or a latch-in connection element.

9. Secondary endoscope according to one of Claims 1 to 8, wherein
the control element (1; 100) can be pivoted to pivot the bending portion (6; 600) and is provided on its proximal side with markings which inform about the pivoting direction of the bending portion (6; 600).

10. Secondary endoscope according to one of Claims 1 to 9, wherein
the catheter tube (5; 500) is attached to the outer body of the handle body (4; 400).

11. Combination of parent endoscope and secondary endoscope, comprising
a parent endoscope (200) having an access connection (2430) for a secondary endoscope; and a secondary endoscope according to one of Claims 1 to 10.

12. Combination of parent endoscope and secondary endoscope, comprising
a parent endoscope (200) having an access connection (2430) for a secondary endoscope on the distal side of a parent endoscope handle (2400); and
a secondary endoscope according to one of Claims 1 to 10 which is releasably mounted on the access connection (2430) and has a secondary endoscope handle (4; 400) and a catheter tube (5; 500); wherein the catheter (5; 500) of the secondary endoscope is guided through a catheter duct (16; 160, 410) of the secondary endoscope handle (4; 400) ;
wherein the distal end of the secondary endoscope handle (4; 400) is releasably mounted on the access connection (2430) of the parent endoscope (200) ;
wherein, in the mounted position, the secondary endoscope is releasably mounted on the parent endoscope handle (2400) on the proximal side of the access connection (2430) of the parent endoscope;
wherein a catheter inlet opening is positioned on the secondary endoscope proximally from the mounting location of the secondary endoscope.

13. Combination according to Claim 12, wherein the secondary endoscope handle (4; 400) furthermore has an additional duct inlet opening (451) on the proximal side, and a fluid inlet opening on the distal side.

## Revendications

1. Endoscope secondaire pouvant se monter sur un endoscope principal, avec
un corps de saisie (4; 400),
un tuyau flexible de cathéter (5; 500) monté sur le corps de saisie (4; 400),
un élément de commande pivotant (1; 100) sur le côté proximal du corps de saisie (4; 400) pour la commande d'une partie flexible (6; 600) disposée à l'extrémité distale du tuyau flexible de cathéter (5; 500),
un raccord de montage sur le côté distal du corps de saisie (4; 400) pour la fixation de l'endoscope secondaire à un endoscope principal (200), dans lequel le raccord de montage est monté sur le corps extérieur du corps de saisie (4; 400) **caractérisé en ce que** le corps de saisie (4; 400) présente un corps extérieur avec un canal intérieur et un corps intérieur (3; 300) disposé dans le canal intérieur du corps extérieur, dans lequel le corps intérieur (3; 300) est déplaçable par rapport au corps extérieur, et **en ce que** l'élément de commande (1; 100) est disposé sur le côté proximal du corps intérieur (3; 300) et peut pivoter par rapport au corps intérieur (3; 300).

2. Endoscope secondaire selon la revendication 1, dans lequel le corps de saisie (4; 400) présente un canal de cathéter (16, 35; 160) pour le guidage du tuyau flexible de cathéter (5; 500).

3. Endoscope secondaire selon la revendication 2, dans lequel l'extrémité proximale de l'élément de commande (1; 100) présente une ouverture d'entrée proximale du canal de cathéter (16, 35; 160).

4. Endoscope secondaire selon l'une quelconque des revendications 1 à 3, dans lequel l'extrémité proximale du tuyau flexible de cathéter (5; 500) est monté sur le côté distal du corps de saisie (4; 400).

5. Endoscope secondaire selon l'une quelconque des revendications 1 à 4, dans lequel l'extrémité proximale du tuyau flexible de cathéter (5; 500) est montée sur le côté extérieur du corps de saisie (4; 400).

6. Endoscope secondaire selon l'une quelconque des revendications 1 à 5, dans lequel l'extrémité distale du corps de saisie (4; 400) présente un corps de montage (1000) pour le montage sur l'endoscope principal, dans lequel le corps de montage (1000) présente une partie du canal de cathéter (16, 35; 160) pour le guidage du tuyau flexible de cathéter (5; 500).

7. Endoscope secondaire selon la revendication 6, dans lequel le corps de montage (1000) est muni d'un marquage, qui informe sur l'état monté ou démonté.

8. Endoscope secondaire selon la revendication 6 ou 7, dans lequel le corps de montage (1000) est un élément de connexion Luer Lock, un élément de fermeture à vis, un élément de fermeture à baïonnette ou un élément de connexion par encliquetage.

9. Endoscope secondaire selon l'une quelconque des revendications 1 à 8, dans lequel l'élément de commande (1; 100) est pivotant pour faire pivoter la partie flexible (6; 600) et est muni sur son côté proximal de marquages, qui informent sur le sens de pivotement de la partie flexible (6; 600).

10. Endoscope secondaire selon l'une quelconque des revendications 1 à 9, dans lequel le tuyau flexible de cathéter (5; 500) est monté sur le corps extérieur du corps de saisie (4; 400).

11. Combinaison d'un endoscope principal et d'un endoscope secondaire, avec
un endoscope principal (200) avec un raccord d'accès (2430) pour un endoscope secondaire; et
un endoscope secondaire selon l'une quelconque des revendications 1 à 10.

12. Combinaison d'un endoscope principal et d'un endoscope secondaire, avec
un endoscope principal (200) avec un raccord d'accès (2430) pour un endoscope secondaire sur le côté distal d'une poignée d'endoscope principal (2400); et
un endoscope secondaire selon l'une quelconque des revendications 1 à 10 monté de façon séparable sur le raccord d'accès (2430), avec une poignée d'endoscope secondaire (4; 400) et un tuyau flexible de cathéter (5; 500);
dans laquelle le cathéter (5; 500) de l'endoscope secondaire est guidé par un canal de cathéter (16; 160, 410) de la poignée d'endoscope secondaire (4; 400);
dans laquelle l'extrémité distale de la poignée d'endoscope secondaire (4; 400) est montée de façon séparable sur le raccord d'accès (2430) de l'endoscope principal (200);
dans laquelle dans la position montée l'endoscope secondaire est monté de façon séparable sur la poignée d'endoscope principal (2400) sur le côté proximal du raccord d'accès (2430) de l'endoscope principal;
dans laquelle une ouverture d'entrée de cathéter est positionnée sur l'endoscope secondaire à proximité du site de montage de l'endoscope secondaire.

13. Combinaison selon la revendication 12, dans laquelle la poignée d'endoscope secondaire (4; 400) présente en outre une ouverture d'entrée de canal supplémentaire (451) sur le côté proximal, et une ouverture d'entrée de fluide sur le côté distal.
